# EUROPÄISCHE PATENTSCHRIFT

(11) **EP 0 420 126 B1**
(45) Veröffentlichungstag und Bekanntmachung des Hinweises auf die Patenterteilung: **14.12.1994**
(21) Anmeldenummer: 90118345.9
(22) Anmeldetag: 24.09.1990
(51) Int. Cl.: A61B 5/14, A61M 5/315, A61M 5/50

(54) **Blutentnahmevorrichtung**
Blood sampling device
Dispositif de prélèvement sanguin

(30) Priorität: 26.09.1989 DE 3932109
(43) Veröffentlichungstag der Anmeldung: 03.04.1991
(73) Patentinhaber: Walter Sarstedt Geräte und Verbrauchsmaterial für Medizin und Wissenschaft, D-51588 Nümbrecht (DE)
(72) Erfinder: Sarstedt, Walter, D-Nümbrecht-Rommelsdorf (DE)

(56) Entgegenhaltungen:
- EP-A- 0 208 975
- DE-C- 643 616
- DE-C- 3 811 973
- US-A- 4 367 738

## Beschreibung

Die Erfindung betrifft eine Blutentnahmevorrichtung nach dem Oberbegriff des Patentanspruchs 1.

Blutentnahmevorrichtungen (siehe z.B. DE-PS 29 48 653, 30 49 503) sollen lediglich der Blutentnahme, nicht jedoch dem Einspritzen irgendwelcher flüssiger Substanzen in die Vene eines Patienten dienen, sind jedoch im allgemeinen dazu geeignet.

Bei Einmalspritzen ist es bereits bekannt (DE-Gbm 88 04 656), eine Mehrfachbenutzung dadurch zu verhindern, daß durch Sperrmittel nur ein einmaliges Aufziehen der Spritze und ein einmaliges Spritzen, nicht jedoch ein zweites Aufziehen von Flüssigkeit möglich ist. Zu diesem Zweck muß jedoch der Spritzenkörper mit einem Sperrklingenring in Form eines reibend innerhalb des Spritzenkörpers verschiebbaren Kolbens versehen sein.

Bei einer weiteren bekannten Einmalspritze (US-PS 48 26 483) ist die Kolbenstange mit mehreren Zahnungen versehen, die mit am Boden der Spritze vorgesehenen federnden Rastnasen zusammenarbeiten. Durch Verdrehen der Kolbenstange um 90° kann einmal ein Aufziehen der Spritze und anschließend ein Ausstoßen der aufgezogenen Flüssigkeit beispielsweise in die Vene eines Patienten herbeigeführt werden. Da ein weiteres Verdrehen der Kolbenstange durch Einrasten der Sperrklinke in eine radiale Ausnehmung der Kolbenstange verhindert wird, kann auch diese Spritze nur zum einmaligen Aufziehen und Ausstoßen von Flüssigkeit verwendet werden.

Weiter ist schon eine Blutabnahmevorrichtung (US-PS 43 70 987) bekannt, deren Kolbenstange mit einer Zahnung und deren hintere Wand mit einer Öffnung zum Durchgang der Kolbenstange und mit einer darin angeordneten Anschlagnase versehen ist. Hierdurch soll eine Festlegung des Kolbens in verschiedenen Auszugsstellungen möglich sein, um im Entnahmerohr ein Vakuum vorbestimmter Stärke aufbauen zu können. Sobald das Vakuum im Entnahmerohr erzeugt ist, kann das rückwärtige angeschärfte Ende einer in der Vene des Patienten steckenden Kanüle durch einen am vorderen Ende des Entnahmerohrs befindlichen elastischen Stopfen hindurchgesteckt werden, wodurch das Vakuum im inneren der Kanüle wirksam wird und Blut aus der Vene des Patienten ins Entnahmerohr saugt.

Bei dieser bekannten Blutabnahmevorrichtung muß jedoch die in der hinteren Wand vorgesehene Öffnung für den Durchgang der Kolbenstange so groß sein, daß die Zahnung an der Kolbenstange und die Anschlagnase außer Eingriff bringbar sind, wodurch es möglich ist, den Kolben auch nach vorn zu verschieben, was zum Ausstoßen des aufgenommenen Blutes in einen anderen Behälter auch beabsichtigt ist. Die bekannte Vorrichtung könnte also auch als Spritze mißbraucht werden.

Die Erfindung geht daher aus von der grundsätzlich auch für die Blutentnahme geeigneten Vorrichtung nach der US-PS 48 26 483, bei der die Kolbenstange in axialer Richtung relativ zum Entnahmerohr so geführt ist, daß nicht durch radiale Bewegungen der Kolbenstange die Sperrmittel außer Eingriff gebracht werden können, sondern nur durch Verdrehung der Kolbenstange.

Ausgehend von einer derartigen Vorrichtung stellt sich die Erfindung die Aufgabe, eine Blutentnahmevorrichtung der eingangsgenannten Gattung zu schaffen, deren Benutzung als Spritze ausgeschlossen ist, damit ein vorsätzlicher Mißbrauch der Blutentnahmevorrichtung als Spritze wirksam unterbunden ist.

Zur Lösung dieser Aufgabe sind die Merkmale des kennzeichnenden Teils des Patentanspruchs 1 vorgesehen.

Eine Bewegung des Kolbens in Richtung zum vorderen Ende des Entnahmerohrs ist äußerstenfalls insoweit möglich, als ein gewisser, jedoch äußerst geringer Weg des Kolbens in dieser Richtung zurückgelegt werden muß, um die beispielsweise aus einer Rastzahnung und einer damit zusammenwirkenden Rastzunge bestehenden Sperrmittel zur Wirkung zu bringen. Die zum Eingreifen der Sperrmittel erforderliche Bewegung des Kolbens nach vorn soll jedoch möglichst gering und insbesondere so gering sein, daß hierbei äußerstenfalls ein Teil des in der Kanüle befindlichen Blutes in die Vene zurückgedrückt wird, nicht jedoch irgendein Teil des Inhalts des Entnahmerohrs selbst.

Eine vorteilhafte Ausführungsform ist so ausgebildet, daß die Sperrmittel zwischen der Kolbenstange und dem hinteren Ende des Entnahmerohres angeordnet sind.

Hierbei kann vorgesehen sein, daß die Sperrmittel aus wenigstens einer entlang der Kolbenstange verlaufenden, an dieser angeordneten Rastzahnung und wenigstens einem damit zusammenwirkenden Sperranschlag am hinteren Ende des Entnahmerohrs besteht. Die Rastzahnung soll dabei so fein sein, daß eine wesentliche Bewegung des Kolbens nach vorn zwecks Eingreifen des Sperranschlages in die Rastzahnung nicht erforderlich ist.

Nach einer ersten Alternative dieser Ausführungsform kann die Ausbildung dabei so sein, daß die Zähne der Rastzahnung unnachgiebig sind und der Sperranschlag eine federnd auf die Rastzahnung zu vorgespannte Sperrklinke ist.

Besonders vorteilhaft ist es jedoch, wenn die Zähne der Rastzahnung sich schräg nach vorn und von der Kolbenstange weg erstreckende Federlappen sind, die mit einer den Sperranschlag darstellenden, quer zur Längsstreckung der Kolbenstange verlaufenden festen Sperrstufe zusammenwirken. Dabei ist die Ausbildung zweckmäßig so, daß die Sperrstufe in einem solchen radialen Abstand von der Kolbenstange beginnt, daß die federnd an die Kolbenstange gedrückten Federlappen durch den Spalt zwischen Kolbenstange und Sperrstufe hindurchtreten können. Eine besonders zuverlässige Sperrung wird erzielt, wenn die Sperrstufe in einem radialen Abstand von ihrem Anfang in eine sich nach hinten erstreckende Wand übergeht, welche den unmittelbar hinter der Sperrstufe geschnappten Federlappen beim Nach-Vorn-Stoßen der Kolbenstange an einer die Sperrwirkung aufhebenden radialen Ausdehnung hindert. Diese Ausbildung ist besonders bei einstückiger Ausbildung des Entnahmerohrendes günstig, weil dann die Anbringung einer federnden Sperrklinke dort problematisch sein könnte. Andererseits lassen sich die federnden Lappen an der Kolbenstange beim Herstellungsvorgang problemlos mit anbringen.

Eine weitere Ausführungsform, die die Anordnung einer Rastzahnung an der Kolbenstange entbehrlich macht, kennzeichnet sich dadurch, daß die Sperrmittel aus einem am Ende des Entnahmerohrs angeordneten, einseitig wirkenden Klemmgesperre und der diesem gegenüberliegenden, vorzugsweise aufgerauhten Oberfläche der Kolbenstange besteht. Das Klemmgesperre ermöglicht eine stetige Festlegung des Kolbens bzw. der Kolbenstange in dem Sinne, daß zum Wirksamwerden der Sperrmittel praktisch überhaupt keine Bewegung des Kolbens bzw. der Kolbenstange nach vorn erforderlich ist.

Zur Schaffung einer baulich besonders bevorzugten Ausführungsform der von vornherein vorhandenen und nicht aufhebbaren Drehsicherung sieht die Erfindung nach einer ersten Alternative vor, daß die Drehsicherung durch Eingreifen der Rastzahnung in eine mit Seitenflanken versehene, relativ zum Entnahmerohr drehfest angeordnete, radiale Ausnehmung gebildet ist oder daß die Kolbenstange einen unrunden Querschnitt aufweist, der durch eine entsprechend unrunde Führungsöffnung am hinteren Ende des Entnahmerohrs hindurchgeführt ist.

Statt einer Drehsicherung kann im Sinne der zweiten Alternative des Anspruches 1 vorgesehen sein, daß die Kolbenstange rundum aufgerauht ist und die Klemmteile rund um die Kolbenstange herum angeordnet sind.

Zum Zwecke einer rationellen Montage der erfindungsgemäßen Blutentnahmevorrichtung im Werk sieht eine bevorzugte Ausführungsform der Erfindung vor, daß der am hinteren Ende des Entnahmerohrs vorgesehene Teil der Sperrmittel in einem in das hintere Ende des Entnahmerohrs einsetzbaren und die Durchgangsöffnung für die Kolbenstange aufweisenden Einsatz vorgesehen ist, welcher im Ende des Entnahmerohrs derart festlegbar ist, daß beim Herausziehen und Einschieben der Kolbenstange aus dem bzw. in das Entnahmerohr der Einsatz in seiner festgelegten Lage verbleibt.

Bevorzugt ist der Einsatz dabei in das hintere Ende des Entnahmerohrs einrastbar, und zwar insbesondere so, daß auch durch Manipulationen mittels eines Werkzeuges der Einsatz nicht mehr ohne Zerstörung aus dem hinteren Ende des Entnahmerohrs entfernt werden kann. Auf diese Weise ist jede mißbräuchliche Benutzung der erfindungsgemäßen Blutentnahmevorrichtung als Spritze wirksam vermieden.

Grundsätzlich genügt es, wenn der Einsatz nur auf einer Seite der Kolbenstange vorgesehen ist. Bevorzugt ist es jedoch vorgesehen, daß der Einsatz sich rund um die Kolbenstange erstreckt.

Um einen derartigen ringförmigen Sperreinsatz im Werk einfach montieren zu können, kann entweder vorgesehen sein, daß der Einsatz in zwei Hälften geteilt und so um die Kolbenstange herumlegbar ist, oder die Ausbildung ist so, daß der Einsatz bei durch ein Werkzeug unwirksam gemachten Sperrmitteln von der Kolbenseite her auf die Kolbenstange aufschiebbar ist, wobei allerdings der Kolben beispielsweise durch Abschrauben zeitweise entfernbar ist.

Eine weitere Möglichkeit der Montage besteht darin, daß die Handhabe oder ein hinteres Endstück der Kolbenstange abnehmbar, insbesondere abschraubbar ist, um vom hinteren Ende der Kolbenstange her den Einsatz auf die Kolbenstange aufsetzen zu können.

Mit besonderem Vorteil wird die Erfindung bei einer Blutentnahmevorrichtung mit einem von einer durchstechbaren Elastikplatte verschlossenen vorderen Ende des Entnahmerohres verwendet, wobei die Elastikplatte bevorzugt in einem Ansatz einer vorn auf das Entnahmerohr aufgeschraubten Schraubkappe vorgesehen ist (DE-C2-29 48 653). Auf den Ansatz wird dann eine Führungshülse mit einer beidseitig angeschärften Kanüle aufgeschoben, wobei das rückwärtige Ende der Kanüle die Elastikplatte durchsticht.

Allgemein kennzeichnet sich ein bevorzugtes Verfahren zum Durchführen einer Vakuumprobe dadurch, daß die bei der Vakuumprobe erforderliche, nach hinten gerichtete Bewegung des Kolbens bei noch nicht in das hintere Ende des Entnahmerohres eingesetztem, jedoch vorzugsweise schon über dem hinteren Ende der Kolbenstange angeordneten Einsatz erfolgt und der Einsatz erst nach dem nach oder während der Vakuumprobe vorgenommenen Vorschieben des Kolbens in seine vordere Endposition in die am hinteren Ende des Entnahmerohrs festgelegte montierte Lage gebracht wird.

Bevorzugt wird die Vakuumprobe so durchgeführt, daß der Kolben zunächst bei entlüftetem vorderen Ende des Entnahmerohres so weit nach vorn verschoben wird, daß der Einsatz gerade noch nicht am hinteren Ende des Entnahmerohrs festgelegt, insbesondere eingerastet wird, daß dann das vordere Ende des Entnahmerohrs dicht verschlossen wird, daß anschließend der Kolben ein Stück zurückgezogen und vorzugsweise wieder losgelassen wird, um seine bei erzeugtem Vakuum mehr oder weniger erfolgende Rückkehr in die Ursprungslage zu überwachen und daß bei erfolgreicher Vakuumprobe der Einsatz am hinteren Ende des Entnahmerohres festgelegt, insbesondere eingerastet wird.

Wenn eine derartige Blutentnahmevorrichtung im Werk hergestellt worden ist, kann die Vakuumprobe auch durch kurzzeitiges Zurückziehen des Kolbens vorgenommen werden. Nach der Vakuumprobe erfolgt durch Bewegen des Kolbens nach vorn ein Entlüften des vor dem Kolben vorgesehenen Raums des Entnahmerohrs, z.B. durch kurzzeitiges Losschrauben einer vorderen Schraubkappe.

Diese Maßnahmen können bei einer erfindungsgemäßen Blutentnahmevorrichtung mit dem Anbringen des Einsatzes kombiniert werden, indem der Einsatz während der Vakuumprobe durch Rückziehen des Kolbens noch nicht in das hintere Ende des Entnahmerohrs eingesetzt ist und vorzugsweise schon über dem hinteren Ende der Kolbenstange angeordnet ist, und indem der Einsatz beim anschließenden Entlüften durch Vorschieben des Kolbens in bzw. auf das hintere Ende des Entnahmerohrs in die montierte Lage gedrückt wird, wobei insbesondere ein Vorsprung an der Kolbenstange oder der hinterste Zahn der Rastzahnung oder die Handhabe am Ende der Kolbenstange die Druckkraft auf den Einsatz überträgt. Das Einschieben des Einsatzes in das hintere Ende des Entnahmerohrs erfolgt also im gleichen Arbeitsgang wie das Entlüften des Innenraumes.

Die Erfindung wird im folgenden beispielsweise anhand der Zeichnung beschrieben; in dieser zeigt:
- Fig. 1: eine teilweise aufgebrochene Seitenansicht einer erfindungsgemäßen Blutentnahmevorrichtung,
- Fig. 2: eine teilweise geschnittene vergrößerte Seitenansicht der vorn auf dem Entnahmerohr nach Fig. 1 angeordneten Schraubkappe mit den daran vorgesehenen Bauteilen,
- Fig. 3: eine etwas vergrößerte teilweise geschnittene Seitenansicht dessen Sperrmittel aufweisenden Bereichs der Blutentnahmevorrichtung nach Fig. 1,
- Fig. 4: einen Schnitt nach Linie IV-IV in Fig. 3,
- Fig. 5: eine zu Fig. 3 analoge teilweise geschnittene Seitenansicht mit Sperrmitteln in Form eines Klemmgesperres und
- Fig. 6: eine zu Fig. 3 ähnliche Ansicht mit einer anderen Ausbildung der Sperrmittel.

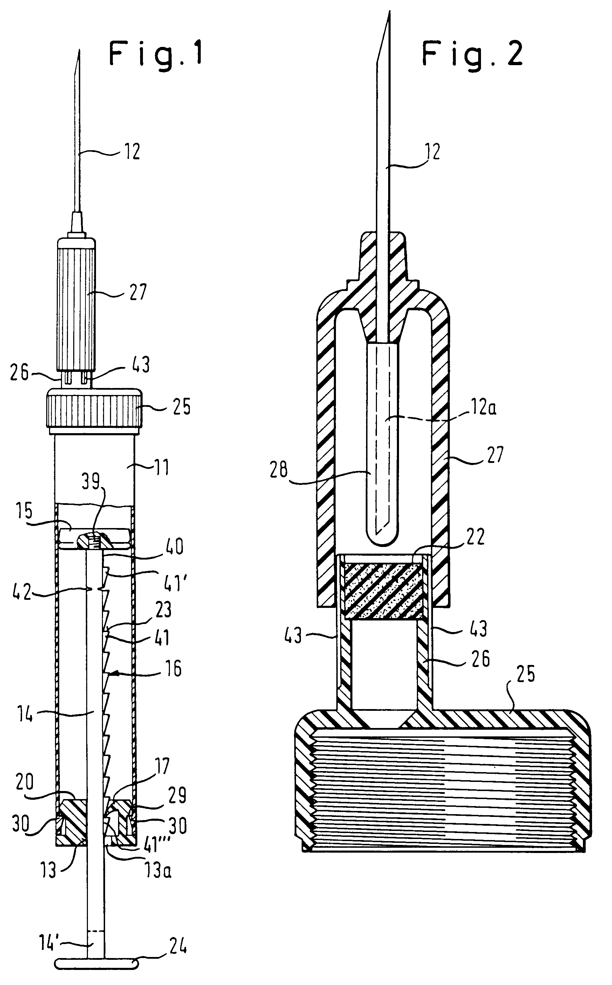

Nach Fig. 1 weist ein kreiszylindrisches Entnahmerohr 11 an seinem vorderen Ende eine auf ein Außengewinde aufgeschraubte Schraubkappe 25 auf, von der sich nach vorn nach den Fig. 1 und 2 ein Ansatz 26 erstreckt, in welchem eine durchstechbare Elastikplatte 22 angeordnet ist. Auf den kreiszylindrischen hohlen Ansatz 26 ist von vorn eine Führungshülse 27 mit kreiszylindrischem Innenraum aufschiebbar, welche an ihrem vorderen Ende eine beidseitig angeschärfte Kanüle 12 trägt, deren hinterer Teil 12a von einem elastischen Schlauch 28 überzogen ist. In der äußeren Mantelfläche des Ansatzes 26 sind axiale Entlüftungsnuten 43 vorgesehen.

Beim Aufschieben der Führungshülse 27 aus der in Fig. 2 wiedergegebenen Position durchsticht das hintere Ende des Kanülenteils 12a den Schlauch 28 und dann die Elastikplatte 22, wodurch eine Verbindung vom vorderen Ende der Kanüle 12 zum Innenraum des Ansatzes 26 und damit zum Innenraum des Entnahmerohrs 11 hergestellt wird. Sobld die Führungshülse 27 wieder vom Ansatz 26 abgezogen und der Teil 12a der Kanüle 12 aus der Elastikplatte 22 herausgezogen wird, legt sich der Schlauch 28 selbsttätig um den hinteren Teil 12a der Kanüle 12 herum, während sich das durch die Elastikplatte 22 gestochene Loch aufgrund der Elastizität der Elastikplatte 22 von selbst wieder schließt.

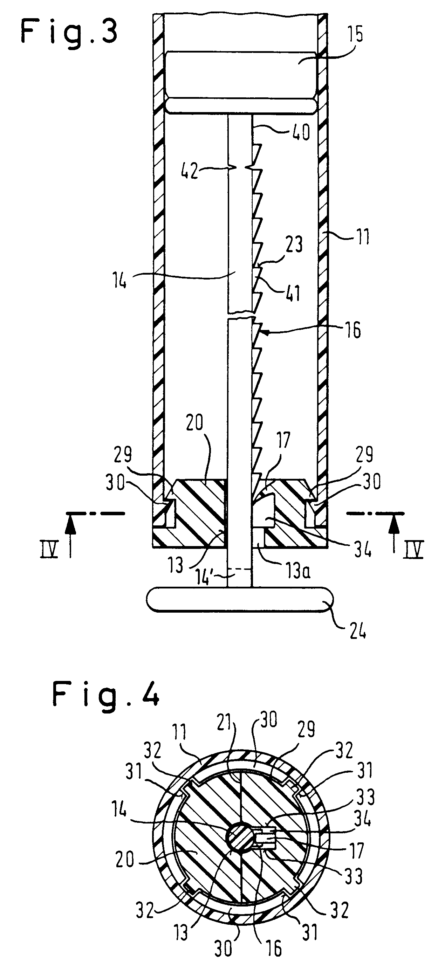

In dem Entnahmerohr 11 ist nach Fig. 1 und 3 ein Kolben 15 axial gleitend und dicht angeordnet, in den bei 39 von hinten eine Kolbenstange 14 eingeschraubt ist, die auf einer Seite gemäß den Fig. 1 und 3 eine sich parallel zur Achse der Kolbenstange 14 erstreckende Rastzahnung 16 trägt, deren Zähne 41 an ihrem vorderen Ende eine sich im wesentlichen senkrecht zur Kolbenstange 14 erstreckende Sperrfläche 23 aufweisen und hinten zur Kolbenstange 14 schräg abfallen.

Nach Fig. 1, 3 und 4 ist in das hintere Ende des Entnahmerohres 11 ein ringförmiger Einsatz 20 eingesetzt, der eine axiale Führungsöffnung 13 für die Kolbenstange 14 aufweist und außerdem mit einer der Rastzahnung 16 gegenüberliegenden federnden Sperrklinke 17 versehen ist. Die Zähne der Rastzahnung 16 und die Sperrklinke 17 sind so geneigt, daß beim Herausziehen der Kolbenstange 14 aus dem Entnahmerohr 11 die Sperrklinke 17 von Zahn zu Zahn schnappen kann, während beim Versuch, die Kolbenstange 14 in Richtung der Schraubkappe 25 zu bewegen, die Sperrklinke 17 gegen die Sperrfläche 23 des gerade dort befindlichen Zahnes 41 der Rastzahnung 16 gedrückt wird, wodurch die Bewegung der Kolbenstange 14 in dieser Richtung gesperrt ist. Die Kolbenstange 14 ist in der Führungsöffnung 13 so geführt, daß sie sich nicht von der Sperrklinke 17 wegbewegen kann. Hierzu ist die Führungsöffnung 13 nach Fig. 1 und 3 so geformt, daß die von der Rastzahnung 16 abgewandte glatte Seitenfläche der Kolbenstange 14 an der Wand der Führungsöffnung 13 anliegt. Diametral gegenüberliegend ist die Durchgangsöffnung bei 13a etwas größer, um axial nach hinten an die Sperrklinke 17 anschließend ausreichend Platz für das Hindurchtreten der Rastzahnung 16 zu schaffen.

Besonders wichtig ist, daß die Sperrklinke 17 in der in Fig. 3 dargestellten Grundposition entspannt ist, sich also nicht in Höhe der Sperrzähne befindet. Auf diese Weise verliert die Sperrklinke 17 ihre Federkraft selbst dann nicht, wenn die Blutentnahmevorrichtung längere Zeit gelagert wird.

Radial außen weist der Einsatz 20 Rastvorsprünge 29 auf, die nach innen vorstehende Gegenrasten 30 am hinteren Ende des Entnahmerohres 11 hintergreifen. Die Rastvorsprünge 29 und die Gegenrasten 30 haben gerade eine umgekehrte Sperrwirkung wie die Rastzahnung 16 und die Sperrklinke 17, d.h., daß der Einsatz 20 in Richtung auf die Schraubkappe 25 von hinten in das Entnahmerohr 11 eingesetzt und dort verrastet werden kann, während ein axiales Herausziehen des Einsatzes 20 nach hinten durch die mit der Gegenrast 30 zusammenwirkenden Rastvorsprünge 29 verhindert wird.

An bestimmten Stellen des Umfanges der Gegenrasten 30 können radiale Einschnitte 31 (Fig. 4) vorgesehen sein, in welche radiale Stege 32 des Einsatzes 20 zwecks Drehsicherung des Einsatzes 20 in dem Entnahmerohr 11 eingreifen.

Eine solche Drehsicherung ist erforderlich, sofern - wie in den Fig. 1, 3 und 4 dargestellt - die Rastzahnung 16 und die Sperrklinke 17 nur auf einer Seite der Kolbenstange 14 vorgesehen sind. Es kann auch jede andere Drehsicherung für den Einsatz 20 verwendet werden, die sicherstellt, daß die Rastzahnung 16 und die Sperrklinke 17 stets in Ausrichtung bleiben.

Damit auch die Kolbenstange 14 relativ zum Einsatz 20 die richtige Drehposition hat, können die Seitenflanken 33 (Fig. 4) einer radialen Ausnehmung 34 des Einsatzes 20, in welcher die Sperrklinke 17 untergebracht ist, in Zusammenwirkung mit der Rastzahnung 16 eine entsprechende Drehsicherung herbeiführen. Erfindungsgemäß sind also der Einsatz 20 relativ vom Entnahmerohr 11 und die Kolbenstange 14 relativ zum Einsatz in vorgegebenen Drehpositionen festgelegt.

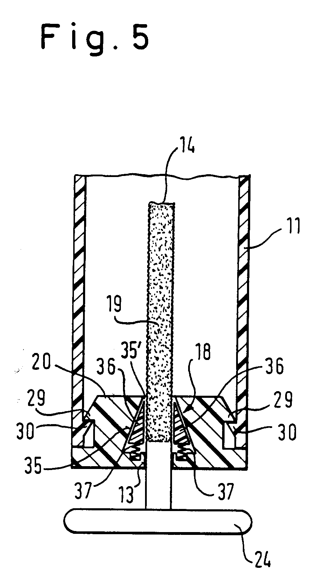

Bei dem Ausführungsbeispiel nach Fig. 5 sind diametral gegenüberliegende Seiten der Kolbenstange 14 mit einer aufgerauhten Oberfläche 19 versehen. In dem Einsatz 20 ist innen ein durch in sich nach vorn verjüngenden keilförmigen Ausnehmungen 35′ vorgesehene komplementäre Keile 36 ausgebildetes Klemmgesperre angeordnet. Die Keile 36 stehen mit ihren radialen Innenflächen im Reibeingriff mit der Oberfläche 19 der Kolbenstange 14, indem sie durch Federn 37 nach vorn verschoben werden. Wird die Kolbenstange 14 von der Schraubkappe 25 weggezogen, so lösen sich die Keile 36 von den sich in Richtung zur Schraubkappe 25 sich verjüngenden Keilflächen 35. Ein Zurückziehen der Kolbenstange 14 ist also möglich.

Wird die Kolbenstange 14 dagegen in Richtung auf die Schhraubkappe 25 zu verschoben, so verkeilen sich die Keile 36 zwischen den Keilflächen 35 des Einsatzes 20 und den Oberflächen 19 der Kolbenstange 14. Dieses Verkeilen unterstützt die Wirkung der Federn 37, welche die Keile 36 von hinten gegen die sich nach vorn verjüngenden Keilflächen 35 drükken. Die Keile 36 verjüngen sich ebenfalls nach vorn und weisen außer dem inneren, an die Fläche der Kolbenstange 14 angepaßten Klemmflächen außen an die konischen Flächen 35 angepaßte Keilflächen auf. Es könnten auch - bei rundum aufgerauhter Kolbenstange 14 - rundum die Kolbenstange 14 Klemmkeile 36 vorgesehen sein.

Die Wirkungweise der beschriebenen Blutentnahmevorrichtungen ist wie folgt:
Bei der Montage im Werk wird der ringförmige Einsatz 20, wenn er einteilig und gemäß Fig. 1, 3 ausgebildet ist, in der weiter unten beschriebenen Weise zunächst auf den hinteren Bereich der Kolbenstange 14 aufgebracht.

Entweder wird dann der Einsatz 20 vor dem Einrasten in das hintere Ende des hintere Ende des Entnahmerohres 11 gemäß Fig. 1 schon über den hintersten Zahn 41‴ geschnappt oder er bleibt auf dem Kolbenstangenstück zwischen dem hinteren Ende der Rastzahnung 16 und der am Ende der Kolbenstange 14 vorgesehenen Handhabe 24.

Der Kolben 15 mit der Kolbenstange 14 und dem daran befindlichen Einsatz 20 wird dann von hinten in das Entnahmerohr 11 eingeschoben, allerdings nur so weit, daß der Einsatz 20 noch aus dem hinteren Ende des Entnahmerohrs 11 hervorsteht und nicht darin eingerastet ist.

Anschließend wird nun die Schraubkappe 25 aufgeschraubt und durch Rückziehen der Kolbenstange 14 die Vakuumprobe vorgenommen, indem der Kolben nach einer bestimmten Rückzugsbewegung losgelassen und geprüft wird, wie weit er sich aufgrund des erzeugten Vakuums wieder nach vorn in seine Ausgangslage zurückbewegt. Liegt irgendwo eine Undichtigkeit vor, so gelangt der Kolben nach Loslassen nicht mehr ganz in seine Ausgangslage zurück, und das betreffende Entnahmerohr kann entweder repariert oder als Ausschuß betrachtet werden. Ist die Vakuumprobe erfolgreich gewesen, so wird der Einsatz 20 so weit nach vorn geschoben, bis er in das hintere Ende des Entnahmerohres 11 eingeschnappt ist. Dies soll zweckmäßig erst erfolgen, wenn der Kolben 15 sich in der vordersten Position im Entnahmerohr 11 befindet. Ist die Sperrklinke 17 schon hinter den hintersten Zahn 41‴ (Fig. 1) geschnappt, kann das Vorschieben des Einsatzes 20 auch durch Vorschieben der Kolbenstange 14 erfolgen, wozu allerdings durch geringfügiges Loschrauben der Schraubkappen 25 eine kurzzeitige Entlüftung herbeigeführt werden sollte. Bevorzugt ist es aber wie gesagt, wenn der Einsatz 20 frei verschiebbar zwischen der Handhabe 24 und der Verzahnung 16 angeordnet ist, denn auf diese Weise kann zunächst der Kolben 15 bei noch abgeschraubter Schraubkappe 25 in die vorderste Position gebracht werden, worauf dann die Schraubkappe 25 aufgeschraubt und die Vakuumprobe in der vorstehend beschriebenen Weise durchgeführt wird. Der Einsatz kann dann bei bereits in seiner endgültigen Position befindlichem Kolben 15 separat in der hintere Ende des Entnahmerohres 11 eingerastet werden.

Bei der Blutentnahme wird dann wie folgt vorgegangen:
Die Führungshülse 27 wird auf den Ansatz 26 (Fig. 2) aufgeschoben, wobei der hintere Teil 12a der Kanüle 12 die Elastikplatte 22 durchsticht. Nunmehr kann die Vene punktiert und durch Rückziehen des Kolbens 15 Blut abgenommen werden. Hierbei schnappt die Sperrklinke 17 nach Fig. 1 von Zahn zu Zahn der Rastzahnung 16. Wird die Handhabe 24 losgelassen oder wird versucht, sie in entgegengesetzter Richtung auf die Schraubkappe 25 zu zu bewegen, so ist dies wegen Einrastens der Sperrklinke 17 in den gerade zugeordneten Zahn 41 der Rastzahnung 16 nicht möglich. Entsprechendes gilt für die Ausführungsform nach Fig. 5, wo die Sperrkeile 36 eine Bewegungsumkehr der Kolbenstange 14 in Richtung auf die Schraubkappe 25 zu verhindern.

Die Erfindung eignet sich auch dazu, vor dem Aufsetzen der Führungshülse 27 auf den Ansatz 26 ein Vakuum durch Zurückziehen der Kolbenstange 14 aufzubauen. Während bei bekannten Blutentnahmevorrichtungen dieser Art der Kolben erst in vollständig zurückgezogenem Zustand in eine Gegenrast des Entnahmerohres 11 einrastet, findet erfindungsgemäß eine Verrastung auch schon bei nur geringfügig zurückgezogenem Kolben 15 statt, so daß der Aufbau des Vakuums in jeder Rückzugsstellung des Kolbens 15 unterbrochen werden kann. Auf diese Weise ist es möglich, auch ein weniger starkes Vakuum, als es bei vollständigem Rückzug des Kolbens 15 erzielt wird, aufzubauen, was z.B. bei Patienten mit schlechten und empfindlichen Venen von Vorteil sein kann. Auf diese Weise kann auch die Menge von bei einer Vakuumentnahme entnommenem Blut in gewünschter Weise begrenzt werden. Ein Zusammenbruch das Vakuums beispielsweise durch bewußtes oder unbewußtes Vorschieben des Kolbens ist dabei aufgrund der erfindungsgemäßen Sperrmittel nicht möglich.

Die Erfindung verhindert also nicht nur ein gewolltes oder ungewolltes Zurückbewegen des Kolbens 15, sondern ermöglicht auch ein genau dosiertes Aufbauen und Halten eines gewünschten Vakuums innerhalb des Entnahmerohrs 11.

Um den Einsatz 20 auf besonders einfache Weise montieren zu können, sofern er gemäß den dargestellten Ausführungsbeispielen rund um die Kolbenstange 14 herumläuft, kann er gemäß Fig. 4 entlang einer axialen Trennungsfläche 21 in zwei Teile geteilt sein, die vor der Montage in geeigneter Weise um die Kolbenstange 14 herumgelegt werden.

Es ist aber auch möglich, die Handhabe 24 (Fig. 1, 3) oder ein hinteres Endstück 14′ der Kolbenstange 14 abzuschrauben, dann von hinten den Einsatz 20 auf das verzahnungsfreie Endteil der Kolbenstange 14 aufzuschieben und anschließend die Handhabe 24 oder das Endstück 14′ wieder anzuschrauben.

Die Kolbenstange kann auch einen unrunden Querschnitt aufweisen, der durch eine entsprechend unrunde Führungsöffnung 13 in dem Einsatz 20 hindurchgeführt ist, wodurch eine einwandfreie Winkellage der Kolbenstange 14 relativ zum Einsatz 20 gewährleistet wird.

Bei vollständig zurückgezogenem Kolben 15 kann die gemäß Fig. 1 bei 39 eingeschraubte Kolbenstange 14 herausgedreht werden, wozu allerdings der Kolben 15 in nicht dargestellter Weise am hinteren Ende des Entnahmerohrs 11 verrastet sein sollte. Außerdem muß der Bereich 40 vor der Rastzahnung 16 querschnittsmäßig so ausgebildet sein, daß bei vollständig zurückgezogenem Kolben 15 eine Verdrehung der Kolbenstange 14 relativ zum feststehenden Kolben 15 möglich ist.

Es ist aber auch möglich, daß der Kolben 15 in seiner am weitesten zurückgezogenen Position dadurch verrastet wird, daß der vorderste Zahn 41′ der Rastzahnung 16 hinter die Sperrklinke 17 schnappt und daß anschließend die Kolbenstange 14 an einer hinter dem ersten Zahn 41′ vorgesehenen Sollbruchstelle 42 abgebrochen wird. Ein und derselbe Zahn 41′ kann somit sowohl zur Vermeidung einer Rückbewegung des Kolbens 15 als auch zum Sichern des Kolbens 15 in der am meisten zurückgezogenen Position verwendet werden.

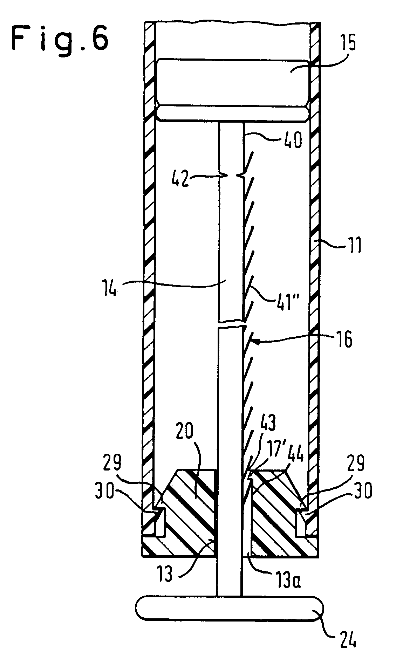

Nach Fig. 6 besteht die an der Kolbenstange 14 vorgesehene Verzahnung 16 aus hintereinander an der Kolbenstange 14 angeordneten Federlappen 41˝, die sich in gleichen Abständen von der Kolbenstange 14 nach vorn und von ihr weg erstrecken. Die Federlappen 41˝ sind drucksteif ausgebildet, können jedoch radial nach innen zur Kolbenstange 14 hin einfedern.

Statt der Sperrklinke 17 nach den Fig. 1 und 3 ist am Einsatz 20 im Bereich der Verzahnung 16 eine in den Freiraum 13a radial nach innen vorspringende starre Ringstufe 17′ vorgesehen, welche zur Kolbenstange 14 einen solchen Abstand 43 aufweist, daß beim Zurückziehen der Kolbenstange 14 die Federlappen 41˝ unter Einfedern durch den dem Abstand 43 entsprechenden Spalt hindurchgelangen können, um schließlich hinter die Ringstufe 17′ zu schnappen, wie das für den hintersten Federlappen in Fig. 6 darsgestellt ist.

Radial außen an das Ende der Ringstufe 17′ schließt sich eine parallel zur Kolbenstange 14 verlaufende und sich nach hinten erstreckende Stutzwand 44 an, an der sich die hinter die Ringstufe 17′ geschnappten Federlappen 41˝ radial von innen anlegen. Auf diese Weise wird beispielsweise beim Vorschieben der Kolbenstange 14 aus der Position nach Fig. 6 ein radiales Ausfedern des hinter die Stufe 17′ eingeschnappten Federlappens vermieden.

Beim Zurückziehen des Kolbens 15 bzw. der Kolbenstange 14 aus der Position nach Fig. 6 schnappen die Federlappen 41˝ sukzessive hinter die starre Ringstufe 17′, und bei einer Bewegungsumkehr verkeilt sich der gerade hinter die Ringstufe 17′ geschnappte Federlappen 41˝ in der Ecke zwischen der Ringstufe 17′ und der Stutzwand 44, so daß eine Bewegungsumkehr auch bei diesem Ausführungsbeispiel unmöglich ist.

## Patentansprüche

1. Blutentnahmevorrichtung mit einem zylinderförmigen Entnahmerohr (11), das ein vorderes, zum Anbringen einer Kanüle (12) bestimmtes Ende und ein eine axiale Führungsöffnung (13) für eine Kolbenstange (14) besitzendes hinteres Ende aufweist, sowie mit einem im Entnahmerohr (11) axial gleitend angeordneten und am vorderen Ende der Kolbenstange (14) bevorzugt lösbar befestigten Kolben (15), wobei über den gesamten Kolbenweg einseitig wirkende Sperrmittel (16, 17; 41˝, 17′; 18, 19) vorgesehen sind, die eine Bewegung des Kolbens (15) im wesentlichen nur in Richtung vom vorderen Ende des Entnahmerohrs (11) nach hinten ermöglichen, eine wesentliche Bewegung in entgegengesetzter Richtung dagegen verhindern, dadurch **gekennzeichnet**, daß ein Außer-Eingriff-Kommen der Sperrmittel (16, 17; 41˝, 17′; 18, 19) durch eine zwischen dem Kolben (15) und dem Entnahmerohr (11) von vornherein vorhandene und unlösbare Drehsicherung (33, 34) oder eine relativ zur Bewegungsrichtung rotationssymmetrische Ausbildung der Sperrmittel (18, 19) unterbunden ist.

2. Vorrichtung nach Anspruch 1, dadurch **gekennzeichnet**, daß die Sperrmittel (16, 17; 18, 19) zwischen der Kolbenstange (14) und dem hinteren Ende des Entnahmerohrs (11) angeordnet sind.

3. Vorrichtung nach Anspruch 2, dadurch **gekennzeichnet**, daß die Sperrmittel aus wenigstens einer entlang der Kolbenstange (14) verlaufenden, an dieser angeordneten Rastzahnung (16) und wenigstens einem damit zusammenwirkenden Sperranschlag (17) am hinteren Ende des Entnahmerohrs (11) besteht.

4. Vorrichtung nach Anspruch 3, dadurch **gekennzeichnet**, daß die Zähne (41) der Rastzahnung (16) unnachgiebig sind und der Sperranschlag (17) eine federnd auf die Rastzahnung (16) zu vorgespannte Sperrklinge ist.

5. Vorrichtung nach Anspruch 3, dadurch **gekennzeichnet**, daß die Zähne (41) der Rastzahnung (16) sich schräg nach vorn und von der Kolbenstange (14) weg erstreckende Federlappen (41˝) sind, die mit einer den Sperranschlag darstellenden, quer zur Längsstreckung der Kolbenstange (14) verlaufenden festen Sperrstufe (17′) zusammenwirken.

6. Vorrichtung nach Anspruch 2, dadurch **gekennzeichnet**, daß die Sperrmittel aus einem am Ende des Entnahmerohres (11) angeordneten, einseitig wirkenden Klemmgesperre (18) bestehend aus Klemmkeilen (36) und der diesem radial gegenüberliegenden, vorzugsweise aufgerauhten Oberfläche (19) der Kolbenstange (14) bestehen.

7. Vorrichtung nach einem der Ansprüche 2 bis 4 dadurch **gekennzeichnet**, daß die Drehsicherung durch Eingreifen der Rastzahnung (16) in eine mit Seitenflanken (33) versehene, relativ zum Entnahmerohr (11) drehfest angeordnete, radiale Ausnehmung (34) gebildet ist.

8. Vorrichtung nach einem der Ansprüche 2 bis 6 dadurch **gekennzeichnet**, daß die Kolbenstange (14) einen unrunden Querschnitt aufweist, der durch eine entsprechend unrunde Führungsöffnung (13) am hinteren Ende des Entnahmerohrs (11) hindurchgeführt ist.

9. Vorrichtung nach Anspruch 6 dadurch **gekennzeichnet**, daß die Kolbenstange (14) rundum aufgerauht ist und die Klemmteile (36) rund um die Kolbenstange (14) herum angeordnet sind.

10. Vorrichtung nach einem der vorhergehenden Ansprüche, dadurch **gekennzeichnet**, daß der am hinteren Ende des Entnahmerohrs (11) vorgesehene Teile (17; 18) der Sperrmittel in einem auf das hintere Ende des Entnahmerohres (11) aufsetzbaren bzw. in dieses einsetzbaren und die Durchgangsöffnung (13) für die Kolbenstange (14) aufweisenden Einsatz (20) vorgesehen ist, welcher im Ende des Entnahmerohrs (11) derart festlegbar ist, daß beim Herausziehen und Einschieben der Kolbenstange aus dem Entnahmerohr (11) bzw. in das Entnahmerohr (11) der Einsatz in seiner festgelegten Lage verbleibt.

11. Vorrichtung nach Anspruch 10, dadurch **gekennzeichnet**, daß der Einsatz (20) am hinteren Ende des Entnahmerohres (11) von hinten einrastbar ist.

12. Vorrichtung nach Anspruch 10 oder 11, dadurch **gekennzeichnet**, daß der Einsatz (20) sich rund um die Kolbenstange (14) erstreckt.

13. Vorrichtung nach Anspruch 12, dadurch **gekennzeichnet**, daß der Einsatz (20) in Axialrichtung in zwei Teile, vorzugsweise zwei Hälften geteilt und so um die Kolbenstange (14) herumlegbar ist.

14. Vorrichtung nach einem der Ansprüche 10 bis 13, dadurch **gekennzeichnet**, daß die Handhabe (14) oder ein hinteres Endstück (14′) der Kolbenstange (14) abnehmbar, insbesondere abschraubbar ist, um vom hinteren Ende der Kolbenstange (14) her den Einsatz (20) auf die Kolbenstange (14) aufsetzen zu können.

15. Verfahren zum Durchführen einer Vakuumprobe bei einer Vorrichtung nach einem der Ansprüche 10 bis 14, dadurch **gekennzeichnet**, daß die bei der Vakuumprobe erforderliche, nach hinten gerichtete Bewegung des Kolbens (15) bei noch nicht in das hintere Ende des Entnahmerohres (11) eingesetztem, jedoch vorzugsweise schon über dem hinteren Ende der Kolbenstange (14) angeordneten Einsatz (20) erfolgt und der Einsatz (20) erst nach dem nach oder während der Vakuumprobe vorgenommenen Vorschieben des Kolbens (15) in seine vordere Endposition in die am hinteren Ende des Entnahmerohrs (11) festgelegte montierte Lage gebracht wird.

16. Verfahren nach Anspruch 15, dadurch **gekennzeichnet**, daß der Kolben (15) zunächst bei entlüftetem vorderen Ende des Entnahmerohres (11) so weit nach vorn verschoben wird, daß der Einsatz (20) gerade noch nicht am hinteren Ende des Entnahmerohrs (11) festgelegt, insbesondere eingerastet wird, daß dann das vordere Ende des Entnahmerohrs (11) dicht verschlossen wird, daß anschließend der Kolben (15) ein Stück zurückgezogen und vorzugsweise wieder losgelassen wird, um seine bei erzeugtem Vakuum mehr oder weniger erfolgende Rückkehr in die Ursprungslage zu überwachen und daß bei erfolgreicher Vakuumprobe der Einsatz (20) am hinteren Ende des Entnahmerohres (11) festgelegt, insbesondere eingerastet wird.

## Claims

1. Blood sample removal device with a cylindrical removal tube (11) which exhibits a front end intended for the attachment of a hollow needle (12) and an axial guidance opening (13) for a rear end provided with a piston rod (14), and with a piston (15) arranged sliding axially in the removal tube (11) and preferably detachably fastened at the front end of the piston rod (14), whereby are provided locking means (16, 17: 41'', 17'; 18, 19) acting unilaterally over the entire piston path, said locking means rendering possible a movement of the piston (15) substantially only from the direction of the front end of the removal tube (11) towards the rear and in contrast hindering any substantial movement in the opposite direction, **wherein** any disengagement of the locking means (16, 17: 41'', 17'; 18, 19) is prevented by a non-detachable anti-rotation device (33, 34) present from the beginning between the piston (15) and the removal tube (11) and or an embodiment of the locking means (18, 19) which is rotationally symmetrical relative to the direction of movement.

2. Device according to claim 1, **wherein** the locking means (16, 17; 18, 19) are arranged between the piston rod (14) and the rear end of the removal tube (11).

3. Device according to claim 2, **wherein** the locking means consist of at least one ratchet toothing (16) extending along the piston rod (14), arranged next to the latter and at least one locking stop (17) cooperating therewith at the rear end of the removal tube (11).

4. Device according to claim 3, **wherein** the teeth (41) of the ratchet toothing (16) are non-yielding and the locking stop (17) is a locking blade resiliently prestressed onto the ratchet toothing (16).

5. Device according to claim 3, **wherein** the teeth (41) of the ratchet toothing (16) are spring tabs (41'') extending obliquely forwards and away from the piston rod (14), said spring tabs cooperating with a fixed locking step (17') representing the locking stop and running transversely to the longitudinal extension of the piston rod (14).

6. Device according to claim 2, **wherein** the locking means consist of a unilaterally acting clamp locking mechanism (18) arranged at the end of the removal tube (11) consisting of clamp wedges (36) and the preferably roughened surface (19) of the piston rod (14) in radially opposite position.

7. Device according to one of the claims 2 to 4, **wherein** the anti-rotation device is embodied by the engagement of the ratchet toothing (16) in a radial recess (34) provided with lateral flanks (33) and arranged torsionally stiffly relative to the removal tube (11).

8. Device according to one of the claims 2 to 6, **wherein** the piston rod (14) exhibits an oval cross-section which is led through a correspondingly oval guidance opening (13) at the rear end of the removal tube (11).

9. Device according to claim 6, **wherein** the piston rod (14) is roughened all round and the clamping components (36) are arranged around the piston rod (14).

10. Device according to one of the aforegoing claims, **wherein** the components (17; 18) of the locking means provided at the rear end of the removal tube (11) are provided with an insert (20) which is detachably mountable at the rear end of the removal tube (11) or insertable into the latter and exhibits the through opening (13) for the piston rod (14), said insert being fixable in the end of the removal tube in such a way that the insert remains in its localized position when the piston rod is pulled out of and inserted into the removal tube (11).

11. Device according to claim 10, **wherein** the insert (20) is engageable from the rear at the rear end of the removal tube (11).

12. Device according to claim 10 or 11, **wherein** the insert (20) extends all round the piston rod (14).

13. Device according to claim 12, **wherein** the insert (20) is divided into two parts, preferably two halves, and is thus able to be laid around the piston rod (14).

14. Device according to one of the claims 10 to 13, **wherein** the handle or a rear end-piece (14) of the piston rod (14) is removable, in particular unscrewable, in order to be able to mount the insert (20) on the piston rod (14) from the rear end of the piston rod (14).

15. Process for the purpose of performing a vacuum test on a device according to one of the claims 10 to 14, **wherein** the rearwards directed movement of the piston (15) required for a vacuum test is effected with the insert (20) not yet inserted into the rear end of the removal tube (11), though preferably already arranged above the rear end of the piston rod (14), and the insert (20) is not brought into the localized mounted position at the rear end of the removal tube (11) until the forward shift of the piston (15) to its front position has been performed after or during the vacuum test.

16. Process according to claim 15, **wherein** the piston (15) initially with the front end of the removal tube (11) vented is pushed far enough forward so that the insert (20) is not quite localized, in particular engaged, at the rear end of the removal tube (11), so that the front end of the removal tube (11) is tightly sealed, so that subsequently the piston (15) is drawn back a little and preferably released again in order to monitor its more or less succeeding return to the original position during the generated vacuum and so that in the event of a successful vacuum test the insert (20) is localized, in particular is engaged, at the rear end of the removal tube (11).

## Revendications

1. Dispositif de prélèvement de sang avec un tube de prélèvement (11) de forme cylindrique présentant une extrémité avant destinée à la mise en place d'une canule (12) et une extrémité arrière possédant une ouverture de guidage axiale (13) pour une tige de piston (14) ainsi qu'un piston (15) à coulissement axial dans le tube de prélèvement (11) et à fixation de préférence amovible à l'extrémité avant de la tige du piston (14), des moyens de blocage (16, 17, 41, 17, 18, 19) à action unilatérale sur toute la course du piston étant prévus pour ne permettre un déplacement du piston (15) essentiellement que dans le sens de l'extrémité avant du tube de prélèvement (11) vers l'arrière, pour empêcher un mouvement dans le sens contraire, caractérisé par le fait qu'un désenclenchement des moyens de blocage (16, 17, 41, 17, 18, 19) est évité par une sécurité à la torsion (33, 34) à priori disponible et indétachable placée entre le piston (15) et le tube de prélèvement (11) ou par une formation des moyens de blocage (18, 19) à symétrie de rotation par rapport au sens du mouvement.

2. Dispositif selon la revendication 1, caractérisé par le fait que les moyens de blocage (16, 17, 18, 19) sont disposés entre la tige du piston (14) et l'extrémité arrière du tube de prélèvement (11).

3. Dispositif selon la revendication 2, caractérisé par le fait que les moyens de blocage consistent au moins en une denture à crans (16) se déplaçant le long de la tige du piston (14) et fixée sur celle-ci et au moins en une butée (17) à action combinée à l'extrémité arrière du tube de prélèvement (11).

4. Dispositif selon la revendication 3, caractérisé par le fait que les dents (41) de la denture à crans (16) sont non flexibles et que la butée (17) est une lame de blocage à action ressort sur la denture à crans (16).

5. Dispositif selon la revendication 3, caractérisé par le fait que les dents (41) de la denture à crans (16) sont à l'oblique vers l'avant et qu'elles consistent en des languettes à ressort (41) écartées de la tige du piston (14) qui agissent avec un palier de blocage fixe (17) représentant la butée et perpendiculaire au sens d'allongement de la tige du piston.

6. Dispositif selon la revendication 2, caractérisé par le fait que les moyens de blocage consistent en un blocage de serrage (18) composé de cales de serrage (36) à action unilatérale et disposé à l'extrémité du tube de prélèvement (11) et en la surface (19) de la tige du piston (14) de préférence rugueuse et radialement opposée au blocage de serrage.

7. Dispositif selon l'une des revendications 2 à 4, caractérisé par le fait que la sécurité à la torsion est obtenue par l'encliquetage de la denture à crans (16) dans un évidement (34) radial à disposition relativement fixe à la rotation par rapport au tube de prélèvement (11) et doté de flancs latéraux (33).

8. Dispositif selon l'une des revendications 2 à 6, caractérisé par le fait que la tige du piston (14) présente une section non circulaire qui se prolonge jusqu'à l'extrémité arrière du tube de prélèvement (11) par une ouverture de guidage (13) non circulaire adéquate.

9. Dispositif selon la revendication 6, caractérisé par le fait que la tige du piston (14) est rugueuse sur son pourtour et que les éléments de serrage (36) sont disposés tout autour de la tige du piston (14).

10. Dispositif selon l'une des revendications précédentes, caractérisé par le fait que les pièces (17, 18) des moyens de blocage prévues à l'extrémité arrière du tube de prélèvement (11) consistent en un embout (20) à mettre en place sur l'extrémité arrière ou dans l'extrémité arrière du tube de prélèvement (11) présentant l'ouverture de passage (13) de la tige du piston (14), cet embout pouvant être bloqué dans l'extrémité du tube de prélèvement (11) de manière à ce qu'il reste dans sa position bloquée lors du dégagement et de l'introduction de la tige du piston du ou dans le tube de prélèvement (11).

11. Dispositif selon la revendication 10, caractérisé par le fait que l'embout (20) est encliquetable par l'arrière sur l'extrémité arrière du tube de prélèvement (11).

12. Dispositif selon la revendication 10 ou 11, caractérisé par le fait que l'embout (20) s'étire tout autour de la tige du piston (14).

13. Dispositif selon la revendication 12, caractérisé par le fait que l'embout (20) est partagé dans le sens axial en deux éléments, de préférence en deux moitiés, qui viennent ainsi s'appliquer autour de la tige du piston (14).

14. Dispositif selon l'une des revendications 10 à 13, caractérisé par le fait que le poussoir (14) ou l'extrémité arrière (14) de la tige du piston (14) est amovible, et notamment dévissable, pour pouvoir mettre en place l'embout (20) sur la tige du piston (14) par l'extrémité arrière de cette dernière.

15. Procédé pour effectuer un prélèvement sous vide sur un dispositif selon l'une des revendications 10 à 14, caractérisé par le fait que le mouvement vers l'arrière du piston (15) nécessaire pour le prélèvement sous vide se fait alors que l'embout (20) ne se trouve pas encore dans l'extrémité arrière du tube de prélèvement (11) mais toutefois, de préférence, déjà en place sur l'extrémité arrière de la tige du piston (14) et que l'embout (20) est amené dans sa position montée à l'extrémité arrière du tube de prélèvement (11) uniquement après le repoussement du piston (15) dans sa position finale avant après ou pendant le prélèvement sous vide.

16. Procédé selon la revendication 15, caractérisé par le fait que le piston (15) est poussé vers l'avant tout d'abord alors que l'extrémité avant du tube de prélèvement (11) est purgée de manière que l'embout (20) se trouve encore a peine sur l'extrémité arrière du tube de prélèvement (11), qu'il soit notamment encliqueté de sorte que l'extrémité avant du tube de prélèvement (11) soit obturée de manière étanche, que, par la suite, le piston (15) soit ramené quelque peu en arrière et de préférence relâché afin de contrôler son retour plus ou moins en cours dans la position initiale sous vide généré et que, lorsque le prélèvement sous vide a été effectué, l'embout (20) soit fixé à l'extrémité arrière du tube de prélèvement (11) et qu'il soit notamment encliqueté.
